# EUROPEAN PATENT SPECIFICATION

(11) **EP 2 209 555 B1**
(45) Date of publication and mention of the grant of the patent: **16.03.2016**
(21) Application number: 08781212.9
(22) Date of filing: 30.06.2008
(51) Int. Cl.: B01L 3/00

(54) **DETECTING AND MIXING IN A CONDUIT IN INTEGRATED BIOANALYSIS SYSTEMS**
DETEKTIEREN UND MISCHEN IN EINER LEITUNG IN INTEGRIERTEN BIOANALYSESYSTEMEN
DÉTECTION ET MÉLANGE DANS UN CONDUIT DANS DES SYSTÈMES DE BIOANALYSE INTÉGRÉS

(30) Priority: 28.06.2007 US 946950 P
(43) Date of publication of application: 28.07.2010
(73) Proprietor: Life Technologies Corporation, Carlsbad, CA 92008 (US)
(72) Inventor: VANN, Charles S., El Granada, California 94018 (US)
(74) Representative: Williams, Richard Andrew Norman
(86) International application number: PCT/US2008/068859
(87) International publication number: WO 2009/006447

(56) References cited:
- EP-A- 1 693 670
- WO-A-2005/039772
- US-A- 5 567 294
- US-A1- 2006 160 162
- US-B1- 6 218 193

## Description

### FIELD

The field of the present disclosure relates to apparatuses and methods for high-throughput detection in integrated bioanalysis systems.

### BACKGROUND

Generally, in bioanalysis, liquid processing is essential for the many process steps involved in obtaining a result. Additionally, many analysis steps, such as sample preparation, reaction, separation, detection, and data processing involved in a broad range of bioanalyses usually require a variety of devices and instrumentation.

For many types of bioanalyses, there is desire to reduce the physical complexity of the biotechnology laboratory and at the same time increase throughput. Therefore, there is a need in the art for bioanalysis systems that can integrate analysis steps such as sample preparation, reaction, separation, detection, and data processing into a single footprint, and at the same time have the flexibility to scale throughput.

The present invention provides a method for luminescent detection as set forth in any of claims 1 to 6. The invention also provides an apparatus as set forth in claim 7.

### BRIEF DESCRIPTION OF THE DRAWINGS

**FIG. 1A** and **FIG. 1B** depict variations of liquid processing manifolds for use in embodiments of integrated bioanalysis, systems.
**FIG. 2A** is a perspective view depicting an integrated bioanalysis system illustrative of the present teachings, and **FIG. 2B** is a cross-section of a side view depicting a subassembly of **FIG. 2A****.**
**FIG. 3A** and **FIG. 3B** are perspective views that depict variations of integrated bioanalysis systems illustrative of the present teachings.
**FIG. 4A** is a perspective view depicting an integrated bioanalysis system illustrative of the present teachings, and **FIG. 4B** is a cross-section of a side view depicting a subassembly of **FIG. 4A****.**
**FIG. 5** depicts a variation of a scanning detection device for use in conjunction with embodiments of liquid processing manifolds.
**FIGS. 6A-6C** depict a method for mixing two liquids using various embodiments of liquid processing manifolds,
**FIGS. 7A-7C** depict a method for mixing two liquids using various embodiments of liquid processing manifolds.

It Is to be understood that the figures are not drawn to scale, nor are the objects in the figures necessarily drawn to scale in relationship to one another. The figures are depictions that are intended to bring clarity and understanding to various embodiments of apparatuses and methods disclosed herein. Wherever possible, the same reference numbers will be used throughout the drawings to refer to the same or like parts.

### DETAILED DESCRIPTION

What is disclosed herein are various embodiments of apparatuses and methods in which luminescent detection is integrated with various analysis steps that are practiced in a range of biological analyses. In bioanalysis, functions such as sample preparation, reaction, and separation require the processing of fluids, such as, for example, the dispensing, mixing, and transport of liquids. Additionally, control of environmental conditions that impact analysis, such as, for example, temperature, pH, and Ionic strength is frequently required. In the various embodiments of apparatuses and methods disclosed herein, detection is integrated with various liquid processing and environmental control functions to create integrated bloanalysls systems thereby. Though the various embodiments of integrated bioanalysis systems are useful for any number of analysis formats, they are adaptable to high-throughput processing of samples.

In disclosed embodiments of apparatuses and methods for integrated bioanalysis systems, liquid processing, environmental control and detection are integrated functions that can be performed in individual conduits. In various embodiments, a plurality of conduits comprises a liquid processing manifold.

The term "conduit" as used herein is any number of liquid processing components known in the art of bioanalysis, such as, but not limited by, tubing, piping, needle, pipette, and pipette tip. Such conduits are useful in a variety of manipulations of samples and reagents for a variety of bioanalyses.

The term "luminescent detection" as used herein includes photoluminescent detection, such as fluorescence and phosphorescence, as well as chemiluminesoent detection, including bioluminescent detection. These types of luminescent detection are useful for a wide range of bioanalyses, offering sensitive detection over a wide range of analytes such as nucleic acids, polypeptides, hormones, drug substances, and the like. An exemplary class of bioanalyses are enabled by a technique know as the polymerase chain reaction (PCR). Some examples of bioanalyses that utilize the PCR technique include viral quantitation, quantitation of gene expression, drug therapy efficacy, DNA damage measurement, pathogen detection, and genotyping.

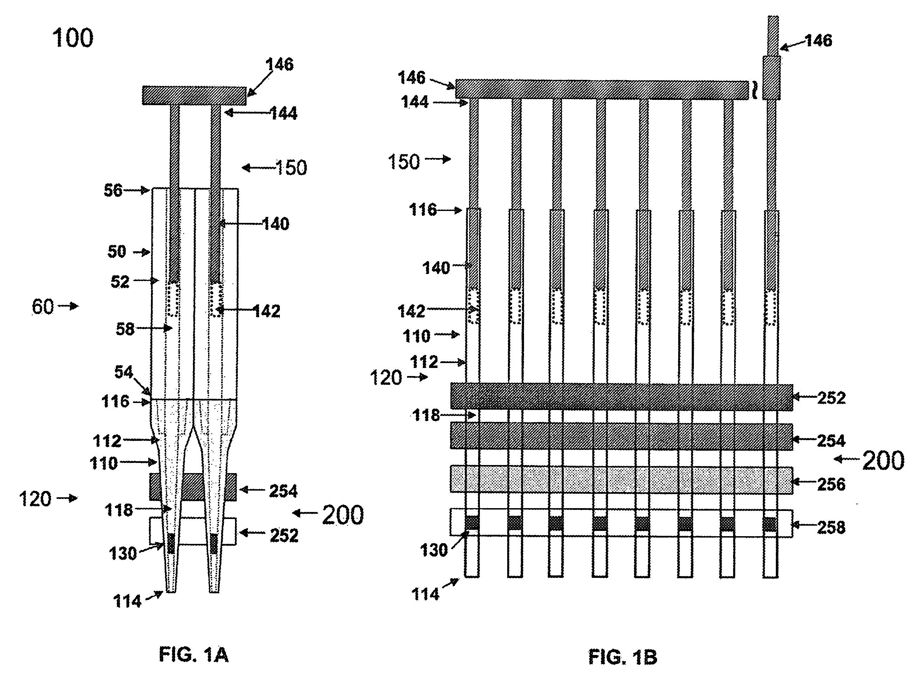

As previously mentioned, in various embodiments of apparatuses and methods for integrated bioanalysis systems, liquid processing, environmental control and detection are integrated functions that can be performed in individual conduits. Additionally, a liquid processing manifold including a plurality of conduits can be useful for high throughput liquid processing systems. The various embodiments of liquid processing manifold **100** depicted in **FIG. 1A** and **FIG. 1B** can be used with embodiments of integrated bioanalysis systems. Liquid processing manifold **100** of FIG. **1A** and **FIG. 1B** can have a conduit assembly **120** having a plurality of conduits **110.** In various embodiments of liquid processing manifold **100** of **FIG. 1A** and **FIG. 1B**, conduit **110** can be removable and replaceable. Conduit **110** has a body **112** which has a first end **114** and a second end **116,** and has a bore **118** extending through body **112.** In some embodiments of liquid processing manifold **100,** conduit assembly **120** can have conduits **110** that are arranged in a linear array. In some embodiments of liquid processing manifold **100,** conduit assembly **120** can have conduits **110** that can be arranged in numerous types of two-dimensional geometries. In some embodiments of liquid processing manifold **100,** conduit **110** can be fabricated from a polymeric material, for example, but not limited by, from classes of polymers such as polypropylene, polyethylene, polyhalohydrocarbon, polycarbonates, and polysilicones, and combinations thereof. In some embodiments of liquid processing manifold **100,** conduit **110** can be fabricated from an inorganic oxide material, for example, but not limited by such as quartz, fused silica, and sapphire, and combinations thereof. In some embodiments of liquid processing manifold **100,** conduit **110** can be fabricated from a metal, such as but not limited by stainless steel, titanium, and combinations thereof. In such embodiments, the metal may be lined with a polymer or inorganic oxide material. In general, attributes for conduits **100** of conduit assembly **120** include, but are not limited by, chemical, mechanical, and thermal stability for their intended use in bioanalysis.

In addition to conduit assembly **120,** various embodiments of liquid processing manifold **100** of **FIG. 1A** and **FIG. 1B** can have a plunger or piston assembly **150** to provide control for processing fluids. In some embodiments of liquid processing manifold **100** of **FIG. 1A****,** piston assembly **150** can function in a housing assembly **60,** that can have a plurality of piston housings **50.** Piston housing **50** has a body **52** having first end **54** and a second end **56,** with a bore **58** extending through body **52.** The second end **116** of conduit **110** can be fitted to first end **54** of the piston housing **50** so that piston housing bore 58 is in fluid communication with conduit bore **118**. Piston assembly **150** can have a plurality of pistons **140.** Piston **140** has a first end **142,** which sealably engages piston housing bore **58** and conduit bore **118,** and a second end **144,** which can be connected to mechanical means for moving the piston **140,** depicted by bar **146** in **FIG. 1A** and **FIG. 1B****.** As indicated in **FIG. 1B**, which shows two variations for bar **146,** mechanical means for moving the piston **140** can be fashioned to move a plurality of pistons, or to move them individually. In **FIG. 1A** and **1** **B,** conduit **110** has first end **114** in which a liquid aliquot or slug **130** can be processed using various embodiments of liquid processing manifolds **100.**

In various embodiments of liquid processing manifold **100** of **FIG. 1A** and **1B**, the movement of piston **140** causes a displacement of fluid in conduit **110,** controlling the movement of fluids in conduit **110** thereby. Such control of fluids may be useful for many types of manipulations of fluids, such as, but not limited by aspiration, mixing, aliquoting, and dispensing, and the like. Moreover, various embodiments of liquid processing manifold **100** enable the processing of a few samples for low throughput processing or many samples for high throughput processing.

In some bioanalyses, piston housing bore **58** and conduit bore **118** of **FIG. 1A** and **1B** may be other than an air/liquid interface for manipulating a liquid aliquot or slug **130** in order to provide an interface tension greater than that provided by an air/liquid interface. In some embodiments of liquid processing manifold **100,** first end **142** of piston **140** may come in direct contact with liquid aliquot or slug **130** to provide a solid/liquid interface. In some variations of liquid processing manifold **100,** the bore-space between first end **142** of piston **140** and liquid aliquot or slug **130** may be partially or totally filled with a fluid that is inert and immiscible and in contact with liquid aliquot or slug **130**, providing a liquid/liquid interface thereby. For example, since the vast majority of bioanalyses are aqueous-based, an example of such an inert, immiscible fluid can be an oil, such as a mineral oil. Additionally, it is desirable that the coefficient of expansion of the inert fluid be low, so as to minimize the impact of the change in volume of the inert fluid when thermostating system **200** is used in variations of integrated bioanalysis system **500.**

In various embodiments of liquid processing manifold **100** of **FIG. 1A** and **1B****,** liquid aliquot or slug **130** positioned at first end **114** of conduit **110** can be finely manipulated and controlled. The phrase "positioned at first end **114"** in reference to position of a liquid aliquot or slug **130** may include embodiments where liquid aliquot or slug **130** can be within the first end, and remains at a position proximal to first end **114,** as well as embodiments where the liquid aliquot or slug **130** can be at least partially extended from first end **114.** In some embodiments, liquid aliquot or slug **130** can be enveloped by an inert, immiscible fluid, such as an oil, for example a mineral oil, so that the protruding liquid can be an oil droplet or film. As will be discussed in more detail subsequently, liquid aliquot or slug **130** can be positioned at first end **114** so that it may be readily detected.

For various disclosed embodiments of integrated bioanalysis system **500,** a thermostating system **200** can be provided to conduit assembly **120** of the liquid processing manifolds **100** by providing one or a plurality of thermostating units, such as for example, thermostating units **252** and **254** of **FIG. 1A** or thermostating units **252, 254, 256** and **258** of **FIG. 1B****.** In addition to the thermostating units, such as **252** and **254** of **FIG. 1A****,** thermostating unit **200** may include additional components, such as thermisters and controllers. The plurality of thermostating units can provide discrete thermal zones for each conduit **110,** which discrete zones may be maintained at a desired temperature. In some embodiments of a thermostating system **200,** the thermostating units may be for example Peltier devices, providing the capability to heat or cool the discrete thermal zones to a desired temperature. In other embodiments of a thermostating system **200** the thermostating units may be, for example, heat blocks that can heat each discrete thermal zones to a desired temperature. An example of an integration of a liquid processing manifold with thermal control for heat cycling during PCR amplification can be found in US 5,985,651 (Hunike-Smith; Nov. 16,1999).

Various embodiments of liquid processing manifolds **100,** fitted with a thermostating system **200** may be incorporated into embodiments of integrated bioanalysis systems. Such systems are integrated to provide a complete range of liquid processing and detection adapted to conduit **110,** so that in addition to liquid processing, the conduit **110** serves as a reaction and detection vessel. Various embodiments of disclosed integrated bioanalysis systems provide flexibility to the end user by providing flexibility in throughput from a few samples to many, flexibility over the volume of liquid aliquot or slug **130** processed by selection of conduit inner diameter and slug length, and flexibility over assay format through selection of automated liquid processing providing control to individual or selected numbers of conduits.

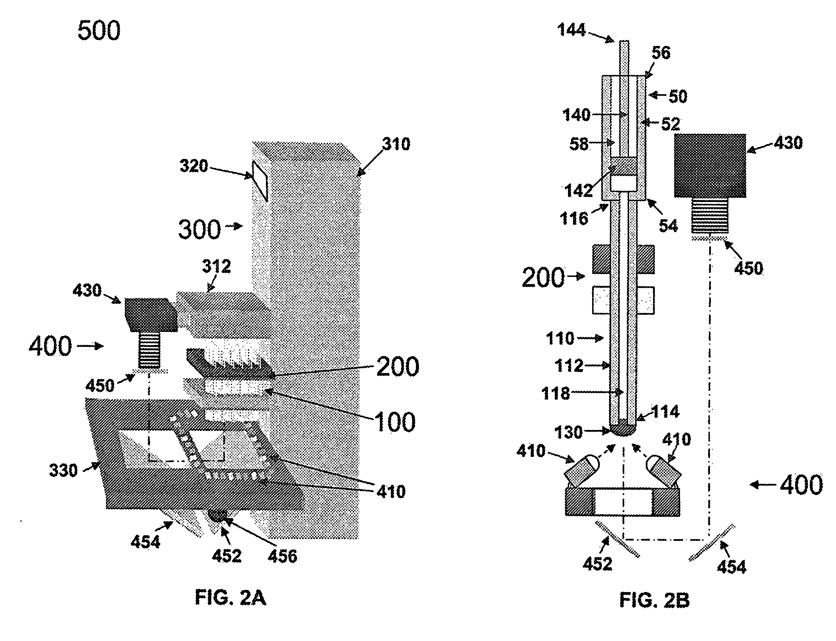

**FIG. 2A** is a perspective view of integrated bioanalysis system **500** according to various embodiments of the present teachings. The integrated bioanalysis system **500** can have instrument support unit **300** which includes instrument support housing **310,** which can be a housing for instrument control system **320.** Additionally, instrument support unit **300** can act as a mount for liquid processing manifold **100** using liquid processing manifold chassis **312,** stage **330,** and detection system **400.** Instrument control system **320** can control the operation of liquid processing manifold 100, control thermostating system **200,** as well as the control the movement of stage **330,** and the operation of detection system **400.** Additionally, instrument control system **320** may provide data processing and report preparation functions. All such instrument control functions may be dedicated locally to the integrated bioanalysis system **500,** or instrument control system **320** may provide remote control of part or all of the control, analysis, and reporting functions.

The detection system **400** of **FIG. 2A** has excitation source **410,** detector **430,** and an optical train including filter **450**, first mirror **452,** second mirror **454,** and motor **456** that can be used to control the position of first mirror **452** and second mirror **454.** According to various embodiments, a detection system can comprise one or more excitation sources, detectors, operational amplifiers, and current control circuits. Such components may have temperature dependent properties, meaning that their properties (e.g., LED intensity) can change with temperature variations, which will be discussed in more detail subsequently. In **FIG. 2A****,** excitation source **410** is depicted as an array of light emitting diodes (LEDs), though different embodiments of detection system **400** may use various excitation sources. An excitation source **410** is used to excite chemical or biochemical species in liquid aliquot or slug **130** positioned at first end **114** of conduit **110,** which first end serves as a reaction and detection vessel. The terms "excitation source," "irradiation source," and "light source" are used in the art interchangeably.

The term "LED" or "light emitting diode" is used herein to refer to conventional light-emitting diodes, i.e., inorganic semiconductor diodes that convert applied electrical energy to light, as well as organic light emitting diode (OLEDs). Conventional LEDs include, for example, aluminum gallium arsenide (AlGaAs), which generally produce red and infrared light, gallium aluminum phosphide, which generally produce green light, gallium arsenide/phosphide (GaAsP), which generally produce red, orange-red, orange, and yellow light, gallium nitride, which generally produce green, pure green (or emerald green), and blue light, gallium phosphide (GaP), which generally produce red, yellow and green light, zinc selenide (ZnSe), which generally produce blue light, indium gallium nitride (InGaN), which generally produce bluish-green and blue light, indium gallium aluminum phosphide, which generally produce orange-red, orange, yellow, and green light, silicon carbide (SiC), which generally produce blue light, diamond, which generally produce ultraviolet light, and silicon (Si), which are under development. LEDs are not limited to narrowband or monochromatic light LEDs; LEDs may also include broad band, multiple band, and generally white light LEDs. Organic LEDs can be polymer-based or small-molecule-based (organic or inorganic), edge emitting diodes (ELED), Thin Film Electroluminescent Device s(TFELD), Quantum dot based inorganic "organic LEDs," and phosphorescent OLED (PHOLED). In addition to LEDs and OLEDs, some embodiments of integrated bioanalysis system **500** may utilized excitation sources such as lasers, for example solid state lasers, such as YAG lasers, gas lasers, such as helium neon (HeNe) lasers, and diode lasers as well as lamps, such as for example, deuterium or mercury lamps.

According to some embodiments of detection system **400** of **FIG. 2A** of integrated bioanalysis system **500,** excitation source **410** can illuminate an entire conduit assembly **120.** In other embodiments, detection system **400,** excitation source **410** can be directed to illuminate portions of first ends **114** of conduit assembly **120** (see **FIG. 1A** and **1B**). An excitation source **410** can include, for example, a combination of two, three, or more LEDs, OLEDs, laser diodes, and the like that are positioned to illuminate all or a portion of conduit assembly **120.** In some embodiments, the LEDs may be white light LEDs that illuminate all or a portion of conduit assembly **120.** In some embodiments, all or a portion of conduit assembly **120** may be illuminated by LEDs having a first relatively short wavelength in the visible range of the electromagnetic spectrum (e.g., UV-blue within the range of 380 nm to 495 nm), a second longer wavelength LED (e.g., green within the range of 450 nm to 495 nm), or a third longer wavelength LED (e.g., red within the range of 620 nm to 750 nm). In various embodiments, excitation source **410** of **FIG. 2A** that illuminates all or a portion of conduit assembly **120** may include combinations of LEDs having different wavelengths in the UV-visible range of the electromagnetic spectrum of between about 380 nm to about 750 nm.

The term "detector" refers to devices that convert electromagnetic energy into an electrical signal, and may include both single element, multi-element and array optical detectors. As previously mentioned, excitation source **410** is used to excite chemical or biochemical species in liquid aliquot or slug **130** positioned at first end **114** of conduit **110.** For the phenomenon of luminescent detection, such excited chemical or biochemical species emit electromagnetic radiation of a longer wavelength than the excitation source. As such, detector **430** is a device capable of monitoring the electromagnetic (e.g., optical) signal from the chemical or biochemical species in liquid aliquot or slug **130** positioned at first end **114** of conduit **110,** providing an electrical output signal or data related to the monitored electromagnetic (e.g., optical) signal. Such devices include, for example, but not limited by photodiodes, including avalanche photodiodes, phototransistors, photoconductive detectors, linear sensor arrays, CCD detectors, CMOS optical detectors (including CMOS array detectors), photomultipliers, and photomultiplier arrays. According to certain embodiments, an optical detector, such as a photodiode or photomultiplier, may contain additional signal conditioning or processing electronics. For example, an optical detector may include at least one preamplifier, electronic filter, or integrating circuit. Suitable preamplifiers include integrating, transimpedance, and current gain (current mirror) pre-amplifiers.

As shown in **FIG. 2A**, detector **430** may be mounted from liquid processing manifold chassis **312,** though detector **430** can be mounted from numerous locations, such as, for example, stage **330** or a free-standing mount, so as to be positioned over second mirror **454.** Detector **430** is shown as a CCD camera, though various embodiments of integrated bioanalysis system **500** of **FIG. 2A** may use a variety of detectors as previously described. Light emitted from conduits **110** of liquid processing manifold **100** is reflected from first mirror **452** to second mirror **454** to be detected by detector **430.** If specificity of the wavelength of electromagnetic energy reaching detector **430** is indicated for some embodiments of integrated bioanalysis system **500,** a filter **450** can be utilized in various embodiments the detection system **400.** Additionally, control system **320** can control motor **456** for adjusting first mirror **452** and second mirror **454,** as well as a motor or motors (not shown) for controlling the positioning of stage **330.** Such control may be important not only for focusing the emitted light from liquid aliquot or slug **130** positioned at first end **114** of conduit **110,** but for other functions, as will be discussed in more detail subsequently.

**FIG. 2B** is a cross-section of a side view depicting a liquid aliquot or slug **130** positioned at first end **114** of conduit **110** using the control of piston **140** and illuminated by excitation source **410,** depicted as LEDs, though as previously described, capable of being a variety of devices. The light emitted by excited chemical or biochemical moieties in liquid aliquot or slug **130** is reflected from first mirror **452** and second mirror **454** to detector **430,** as indicated by the hatched line. As previously discussed, the phrase "positioned at first end **114"** in reference to position of liquid aliquot or slug **130** for the purpose of detection may include embodiments where liquid aliquot or slug **130** can be within the first end, and remains at a position proximal to first end 114, as well as embodiments where liquid aliquot or slug 130 can be at least partially extended from first end 114, as depicted in FIG. 2B. In some embodiments, liquid aliquot or slug 130 can be enveloped by an inert, immiscible fluid, such as an oil, for example a mineral oil, so that the protruding liquid is an oil droplet or film. Most importantly, liquid aliquot or slug 130 can be positioned at first end 114 so that it may be readily detected by detector 430.

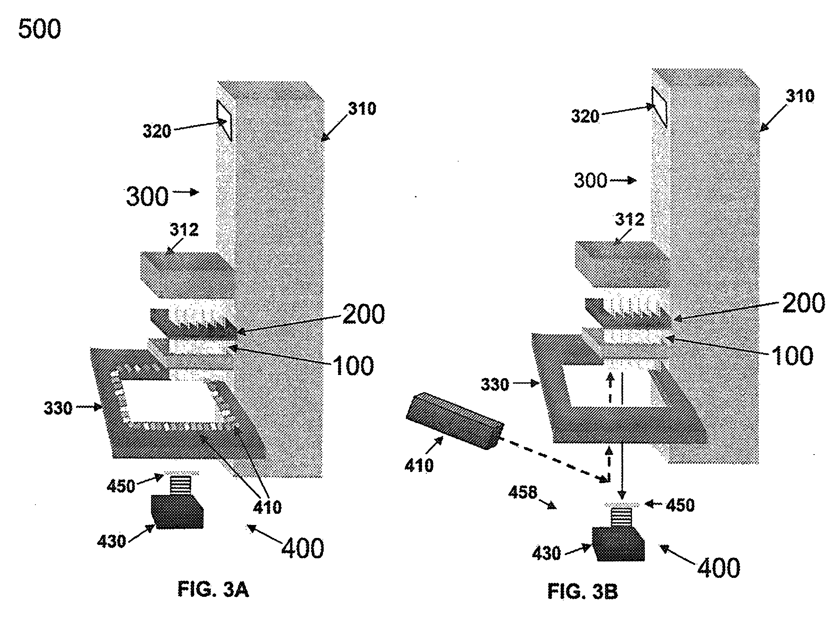

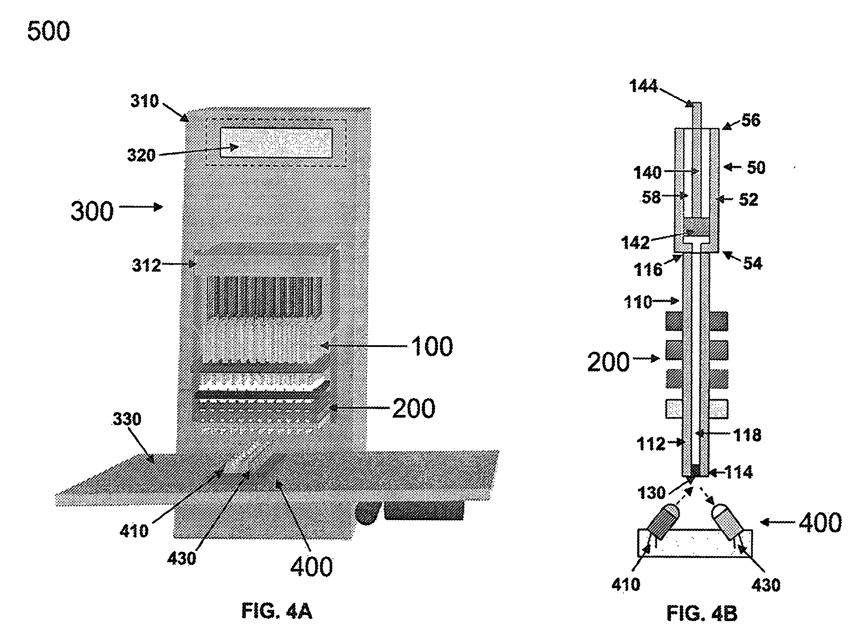

Additional designs of detection systems for integrated bioanalysis system 500 are illustrated by various embodiments of detection system **400** of **FIG. 3A** and **FIG**. **3B**, as well as by various embodiments of detection system **400** of **FIG. 4A** and **FIG. 4B**. Various embodiments of detection system **400** of **FIG. 3A** utilize direct detection of light emitted from excited chemical or biochemical species in liquid aliquots or slugs **130** positioned at first ends **114** of conduit assembly **120** (see **FIG. 1A** and **1B**) by positioning detector **430** directly in view of first ends **114.** Various embodiments of detection system **400** indicated by **FIG. 3B** utilize a dichroic filter **458.** Such filters can be selected to reflect light of specific wavelength range to excite chemical or biochemical moieties in liquid aliquot or slug **130** positioned at first end **114** of conduit **110,** and then pass the emitted light from first end **114** to detector **430.** In FIG. 4A, detection system **400** can be positioned on stage **330.** In some embodiments of integrated bioanalysis system **500** of **FIG. 4A****,** detection system **400** can be attached to stage **330,** and stage **330** can move detection system **400** into position to detect all or a subset of first ends **114** of conduit assembly **120.** In other embodiments of integrated bioanalysis system **500** of **FIG. 4A****,** detection system **400** can be moved along stage **330** to position detection system **400** to detect all or a subset of the first ends **114** of conduit assembly **120.**

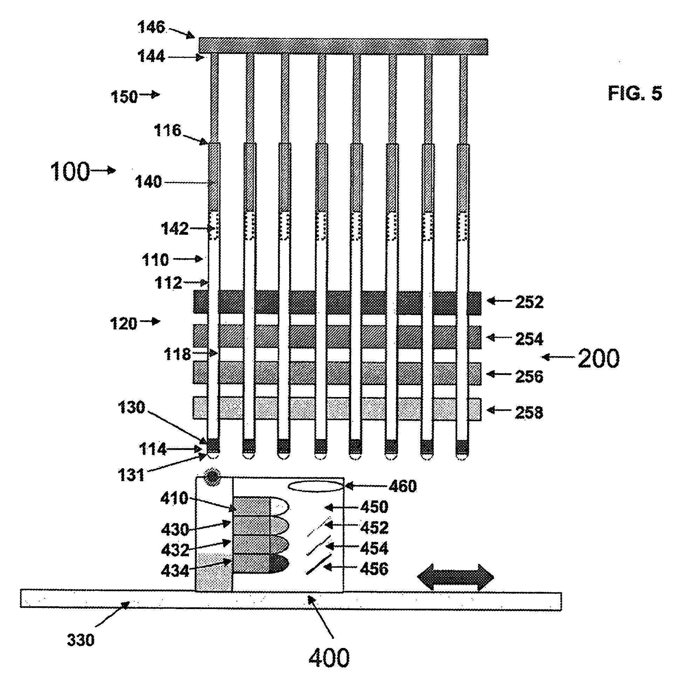

Various embodiments of detection system **400** of **FIG. 4B** utilize of two, three, or more LEDs, OLEDs, laser diodes, and the like that are positioned to illuminate all or a subset of the first ends **114** of conduit assembly **120** and have additionally two, three, or more detecting devices such as photodiodes, phototransistors, photoconductive detectors, linear sensor arrays, such as CMOS array detectors positioned to detect the light emitted by chemical or biochemical moieties in liquid aliquots or slugs **130** for all or a subset of first ends **114** of conduit assembly **120** (see **FIG. 1A** and **1B**). Embodiments of integrated bioanalysis system **500** that can utilize various embodiments of detection system **400** of **FIG. 5** are exemplary of a detection system that can be positioned and moved either along stage **330** or using stage **330.** For some embodiments of a movable detection system **400** of **FIG. 5** at least one excitation source, such as **430, 432,** and **434,** as well as at least one detector **410,** and at least one dichroic filter, such as **450, 452, 454,** and **456** can be used. Additionally, other optical elements, such as a focusing lens **460** may be incorporated in some embodiments of a movable detection system **400** of **FIG. 5****.** An example of a detection system adaptable to embodiments of detection system **400** of **FIG. 5** can be found in US 2006/0121602 (Hoshizaki, et al.; Jun. 8, 2006).

According to the various embodiments of a detection system **400** given in the above, such detection systems can comprise one or more excitation sources **410,** such as LEDs, OLEDs, laser diodes, lasers, lamps, and the like, as well as one or more detectors **430,** such as photodiodes, CCD detectors, and CMOS optical detectors, and the like. Additionally, optical systems may include operational amplifiers, and LED-current control circuits. Such components may have temperature dependent properties, meaning that their properties (e.g., LED intensity) can change with temperature variations. In that regard, variations of detection systems **400** for use with embodiments of integrated bioanalysis systems **500** may utilize a temperature compensation system that can, for example, maintain some or all of these components at a constant temperature to eliminate or reduce changes in the temperature dependent property or properties. The temperature dependent property may also include properties that are a derived or indirect function of a temperature dependent property. Thus, for example, if electrical resistance is a temperature dependent property, current or voltage, which would be functions of the resistance, could also be temperature dependent properties. Other temperature dependent properties may include, for example, temperature dependent properties of an optical detector, such as a photodiode. For example, the "dark current" or noise of a detector may be temperature dependent. Temperature sensors may thus include electronic circuits and signal measurement devices or elements configured to monitor, for example, dark current or noise.

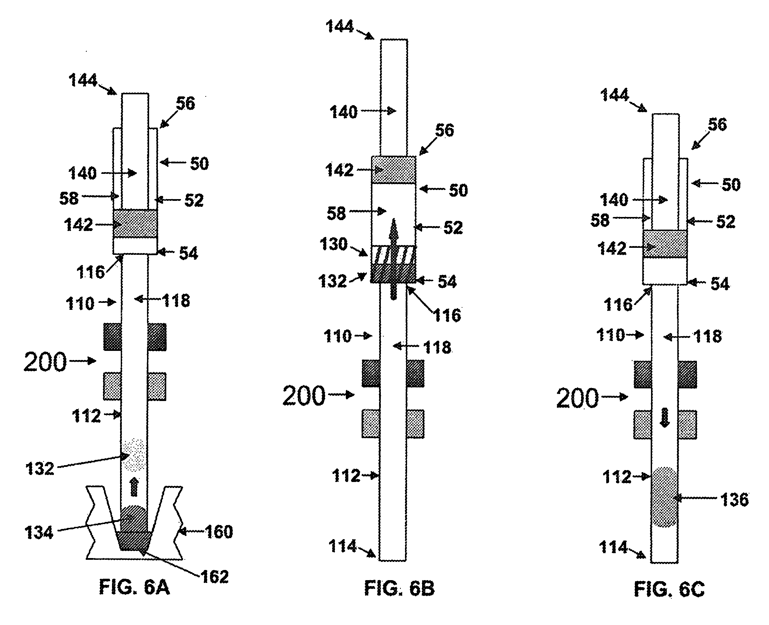

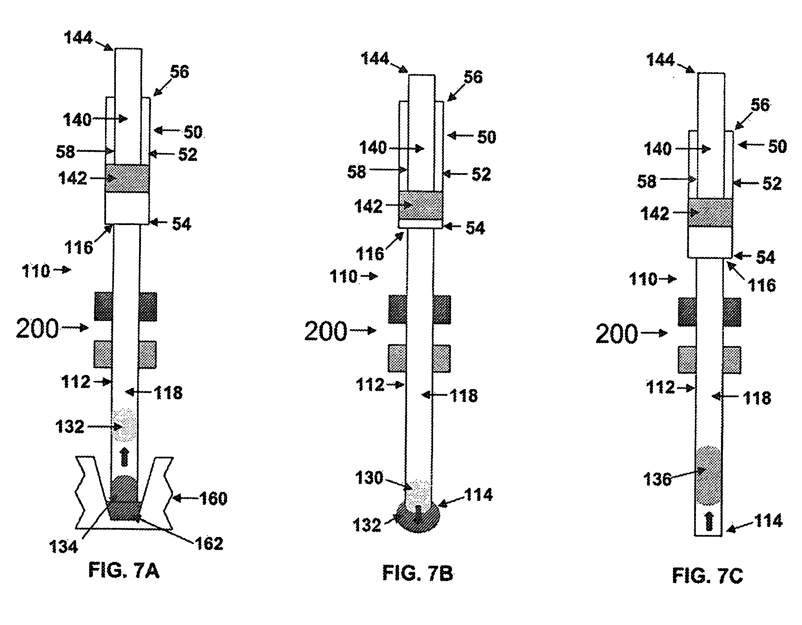

Liquid processing manifolds, such as various embodiments of disclosed liquid processing manifold 100, process liquids taken from samples and reagents held in containing means, for example, but not limited by microtiter plates, as well as various containers such as, but not limited by, vials, tubes, ampoules, and cuvettes, and the like, that are held in holders, such as racks. As one of ordinary skill in the art is apprised, many high-throughput bioanalyses are adapted to a microtiter plate format, for example based on a 8 by 12 array of wells, yielding 96 wells per plate, or higher orders of wells per plate based on a multiple of the 96 well pattern. In a typical operation, liquid processing manifold **100** is used primarily for the dispensing of fluids, while the bioanalysis steps of reacting and detecting are done in containing means. Mixing a reagent or reagents with a sample is necessary to the step of reacting. In that regard; though not of the invention, various methods for on-conduit mixing of a plurality of liquids using embodiments of liquid processing manifold **100,** enabling on-conduit reactions thereby are depicted in **FIGS. 6A, 6B,** and **6C** and **FIGS. 7A, 7B,** and **7C****.**

In methods depicted by **FIGS. 6A, 6B,** and **6C****,** first liquid slug **132** and second slug **134** can be drawn into conduit **110** from a containing means, such as **160,** in which the sample or reagent, such as **162,** has been dispensed **(****FIG. 6A****).** As depicted, first slug 132 and second slug 134 are separated by a segment of another fluid with which they are both immiscible, e.g., air. First slug **132** and second slug **134** can be drawn through conduit **110** and as depicted In **FIG. 6B****,** into a second, wider bore, e.g., piston housing bore **58,** using piston **140.** In methods depicted by **FIGS. 6A, 6B,** and **6C****,** piston housing bore **58** has a diameter that is different than that of conduit bore **118.** In **FIGS. 6B,** and **6C**, as slugs **132** and **134** are drawn first into piston housing bore **58** and then moved back into conduit bore **118,** they are mixed to form mixed slug **136.** The mixing of the first fluid and the second fluid can be increased by drawing mixed slug **136** into the second, wider bore and moving it back again into conduit bore **118.** Other methods of mixing a plurality of slugs based on the difference in bore diameter of a conduit, housing, or combination thereof, can utilize, for example, a tapered conduit, housing or combination thereof. Methods for mixing a plurality of liquid slugs depicted in **FIGS. 7A, 7B,** and **7C** utilize the movement of liquid slugs between conduit bore **118** and first end **114** for on-condult mixing of a plurality of liquid slugs. In **FIG. 7A****,** a first liquid slug **132** can be drawn into conduit **110** from a containing means, such as **160,** in which the sample or reagent, such as **162,** has been dispensed. A second slug **134** can be drawn into first end **114** of conduit **110** as depicted in **FIG. 7B****.** Using piston **140**, first slug **132** and second slug **134** can be drawn up into conduit bore **118** as depicted in **FIG. 7C****,** and then a portion of the combined first slug **132** and second slug 134 can be controllably exuded at first end **114** as depicted in **FIG. 7B****,** effecting the mixing of first slug **132** and second slug **134** thereby to form mixed slug **136.** Though the methods depicted by **FIGS. 6A, 6B,** and **6C** and **FIGS. 7A, 7B,** and **7C** have been demonstrated with a first and second slug, variations of the methods can be extended to mixing higher orders of liquid slugs for numerous samples and reagents. In addition to mixing, other benefits may be realized in the use of various on-conduit manipulations of liquid aliquots or slugs. For example, sample preparation steps, such as, but not limited by, nucleic acid shearing may be done on-conduit.

As previously mentioned, an exemplary class of bioanalyses are enabled by a technique know as the polymerase chain reaction (PCR). One type of PCR reaction is known to those skilled in the art as real-time PCR, which has become a widely used in bioanalyses. An example of a system and method for real time PCR amplification can be found in US 5,928, 907 (Woudenberg, et al.; Jul. 27,1999). A range of embodiments of real-time PCR methods can be performed using various embodiments of an integrated bioanalysis systems 500, as indicated by **FIG. 4A, FIG. 4B** and **FIG. 5****.** In **FIG. 5** conduit bore **118** can be at least partially filled with an oil, such as a mineral oil. Sample and reagents for conducting a quantitative PCR method have been mixed according to variations of methods for on-conduit mixing previously described, and can be formed as slug **130,** which can be thermocycled, i.e., taken through a plurality of thermal cycles, using thermal system **200** for the purpose of amplification of targeted nucleic acid species.

Some embodiments of thermal system **200** of **FIG. 5** can have between about 2 heating blocks to about 4 heating blocks, each of which are controlled to a targeted temperature to create a separate targeted heat zone in conduit **110.** In some embodiments of a quantitative PCR method, a thermal setting of about 95 °C can be maintained for heating block **252,** a thermal setting of about 109 °C can be maintained for heating block **254,** a thermal setting of about 47 °C can be maintained for heating block **256,** and a thermal setting of about 60 °C can be maintained for heating block **258.** In some embodiments of apparatuses and methods for an integrated bioanalysis system **500,** in order to decrease the cycle time, pairing heating blocks for the denaturation portion of the real-time PCR cycle and the extension/annealing portion of the real-time PCR cycle can be done. For example, for the denaturation portion of a PCR cycle, slug **130** can be moved into a thermal zone of about 109 °C of heating block **254** until the desired temperature for slug **130** of about 95 °C is reached, and then slug **130** can be moved into a thermal zone of about 95 °C of heating block **252** for the duration of the denaturation portion of the cycle. Similarly, during the extensionlannealing portion of a PCR cycle, slug **130** can be moved into a thermal zone of about 47 °C of heating block **256** until the desired temperature for slug **130** of about 60 °C is reached, and then slug **130** can be moved into a thermal zone of about 60 °C of heating block **258** for the duration of the extension/annealing portion of the cycle. After each cycle, slug **130** is either in position at first end **114** for detection, or can be readily positioned at first end **114** for detection before the next cycle is initiated. As previously discussed, the phrase "positioned at first end **114"** in reference to position of a liquid aliquot or slug **130** may include embodiments where liquid aliquot or slug **130** can be within the first end, and remains at a position proximal to first end **114,** as well as embodiments where liquid aliquot or slug **130** can be at least partially extended from first end **114.** In some embodiments, liquid aliquot or slug **130** can be enveloped by an inert, immiscible fluid, such as an oil, for example a mineral oil, so that the protruding liquid can be an oil droplet or film **131,** as depicted in **FIG. 5****.**

Though various embodiments of detection system **400** have been illustrated In various embodiments of figures presented, it is recognized by one of ordinary skill in the art that detection of slug **130** can be done on conduit **110** at a location other than the first end **114.** For example, detection of slug **130** could be done in any location along conduit **110** using, for example, fiber optic cables both from an excitation source and to a detector.

The principles of luminescent detection in integrated bioanalysis systems have been described in connection with exemplary embodiments. Accordingly, it should be understood that these descriptions are made for the purpose of illustration, and are not intended to limit the scope of the disclosure. In that regard, what is disclosed herein is not intended to be exhaustive or to limit the illustrations and descriptions to the precise forms depicted. Many modifications and variations will be apparent to the practitioner skilled in the art. What is disclosed was chosen and described in order to best explain the principles and practical application of the disclosed embodiments of the art described, thereby enabling others skilled in the art to understand the various embodiments and various modifications that are suited to the particular use contemplated.

## Claims

1. A method for luminescent detection comprising:
providing a first conduit having a first end and a second end, said second end in fluid communication with fluid control means;
forming with the fluid control means a pendant drop at the first end of the first conduit;
selecting at least one excitation source, the at least one excitation source positioned proximal to the pendant drop, thereby creating at least one selected excitation source;
illuminating the pendant drop with the at least one selected excitation source to excite chemical or biochemical species present in the pendant drop; and
detecting with a detection system light emitted from excited chemical or biochemical species present in the pendant drop.

2. The method for luminescent detection of claim 1 wherein the light emitted is fluorescence.

3. The method for luminescent detection of claim 1 wherein the light emitted is phosphorescence.

4. The method for luminescent detection of claim 1 wherein the light emitted is chemiluminescence.

5. The method of luminescent detection of claim 1, further comprising:
thermocycling a liquid aliquot in the first conduit to produce chemical or biochemical species therein prior to forming the pendant drop with the liquid aliquot.

6. The method of luminescent detection of claim 1, further comprising: amplifying targeted nucleic acid species within a liquid aliquot in the first conduit prior to forming the pendant drop with the liquid aliquot.

7. An apparatus comprising:
a first conduit having a first end and a second end, the second end in fluid communication with fluid control means, wherein the fluid control means is capable of forming a pendant drop at the first end of the first conduit;
at least one excitation source proximal to the first end of the first conduit, wherein the pendant drop at the first end of the first conduit is illuminated by the excitation source; and
a detection system, wherein light emitted from the pendant drop is detected by the detection system.

## Patentansprüche

1. Verfahren zum Lumineszenznachweis, wobei das Verfahren folgendes umfasst:
Bereitstellen einer ersten Leitung mit einem ersten Ende und einem zweiten Ende, wobei sich das genannte zweite Ende in Fluidkommunikation mit einer Fluidregelungseinrichtung befindet;
Bilden mit der Fluidregelungseinrichtung eines Hängetropfens an dem ersten Ende der ersten Leitung;
Auswählen wenigstens einer Erregungsquelle, wobei die wenigstens eine Erregungsquelle proximal zu dem Hängetropfen positioniert ist, wodurch wenigstens eine ausgewählte Erregungsquelle erzeugt wird;
Beleuchten den Hängetropfen mit der wenigstens einen ausgewählten Erregungsquelle, um in dem Hängetropfen vorhandene chemische oder biochemische Spezies zu erregen; und
Nachweisen eines Nachweissystemlichts, das durch in dem Hängetropfen vorhandene chemische oder biochemische Spezies emittiert wird.

2. Verfahren zum Lumineszenznachweis nach Anspruch 1, wobei das emittierte Licht Fluoreszenz ist.

3. Verfahren zum Lumineszenznachweis nach Anspruch 1, wobei das emittierte Licht Phosphoreszenz ist.

4. Verfahren zum Lumineszenznachweis nach Anspruch 1, wobei das emittierte Licht Chemilumineszenz ist.

5. Verfahren zum Lumineszenznachweis nach Anspruch 1, wobei das Verfahren ferner folgendes umfasst:
Thermocycling eines flüssigen Aliquots in der ersten Leitung, um darin vor der Bildung des Hängetropfens mit dem flüssigen Aliquot chemische oder biochemische Spezies zu erzeugen.

6. Verfahren zum Lumineszenznachweis nach Anspruch 1, wobei das Verfahren ferner folgendes umfasst:
Verstärken von Ziel-Nukleinsäurespezies mit einem flüssigen Aliquot in der ersten Leitung vor der Bildung des Hängetropfens mit dem flüssigen Aliquot.

7. Vorrichtung, die folgendes umfasst:
eine ersten Leitung mit einem ersten Ende und einem zweiten Ende, wobei sich das zweite Ende in Fluidkommunikation mit einer Fluidregelungseinrichtung befindet; wobei die Fluidregelungseinrichtung einen Hängetropfen an dem ersten Ende der ersten Leitung bilden kann;
wenigstens eine Erregungsquelle, die proximal zu dem ersten Ende der ersten Leitung ist, wobei der Hängetropfen an dem ersten Ende der ersten Leitung durch die Erregungsquelle beleuchtet wird; und
Nachweissystem, wobei das von dem Hängetropfen emittierte Licht durch das Nachweissystem nachgewiesen wird.

## Revendications

1. Procédé pour la détection luminescente comprenant les étapes consistant à :
fournir un premier conduit possédant une première extrémité et une seconde extrémité, ladite seconde extrémité en communication fluidique avec des moyens de contrôle du fluide ;
former avec les moyens de contrôle du fluide une goutte pendante à la première extrémité du premier conduit ;
sélectionner au moins une source d'excitation, l'au moins une source située à proximité de la goutte pendants, créant ainsi au moins une source d'excitation choisie ;
illuminer la goutte pendante avec l'au moins une source d'excitation choisie pour exciter les espèces chimiques ou biochimiques présentes dans la goutte pendante ; et
détecter à l'aide d'un système de détection de la lumière émise à partir des espèces chimiques ou biochimiques excitées présentes dans la goutte pendant.

2. Procédé pour la détection luminescente selon la revendication 1, dans lequel la lumière émise est de la fluorescence.

3. Procédé pour la détection luminescente selon la revendication 1, dans lequel la lumière émise est de la phosphorescence.

4. Procédé pour la détection luminescente selon la revendication 1, dans lequel la lumière émise est de la chimioluminescence.

5. Procédé pour la détection luminescente selon la revendication 1, comprenant en outre l'étape consistant à :
effectuer le thermocyclage d'un aliquote liquide dans un premier conduit pour produire des espèces chimiques ou biochimiques au sein de celui-ci avant de former la goutte pendante avec l'aliquote liquide.

6. Procédé pour la détection luminescente selon la revendication 1, comprenant en outre l'étape consistant à :
amplifier les espèces d'acides nucléiques ciblées au sein d'un aliquote liquide dans le premier conduit avant de former la goutte pendante avec l'aliquote liquide.

7. Appareil comprenant :
un premier conduit possédant une première extrémité et une seconde extrémité, la seconde extrémité en communication fluidique avec des moyens de contrôle du fluide, dans lequel les moyens de contrôle du fluide sont capables de former une goutte pendante à la première extrémité du premier conduit ;
au moins une source d'excitation à proximité de la première extrémité du premier conduit, dans lequel la goutte pendante à la première extrémité du premier conduit est illuminée par la source d'excitation ;
un système de détection, dans lequel la lumière émise à partir de la goutte pendante est détectée par le système de détection.
